# EUROPEAN PATENT SPECIFICATION

(11) **EP 1 700 597 B1**
(45) Date of publication and mention of the grant of the patent: **24.02.2010**
(21) Application number: 05398003.3
(22) Date of filing: 04.03.2005
(51) Int. Cl.: A61K 31/155, A61K 31/122, A61K 31/164, A61K 9/107, A61P 1/00

(54) **Pharmaceutical composition containing in association ubidecarenone, dexpanthenol and chlorhexidine or a pharmaceutically acceptable salt thereof for cutaneous application**
Pharmazeutische Zusammensetzung enthaltend in Assoziation Ubidecarenon, Dexpanthenol und Chlorhexidine oder eines seiner pharmazeutischen unbedenklichen Salze zur Anwendung auf der Haut
Composition pharmaceutique comprenant en association ubidecarenone, dexpanthenol et chlorhexidine ou un de ses sels pharmaceutiquement acceptables pour application cutanée

(43) Date of publication of application: 13.09.2006
(73) Proprietor: TECNIMEDE-SOCIEDADE TECNICO-MEDICINAL, S.A., 2685-338 Prior Velho (PT)
(72) Inventor: Veiga Abreu Rocha, Alexandre Miguel, Dr., 3050-208 Luso (PT); Pardal Filipe, Augusto Eugénio, Dr., 1800-331 Lisboa (PT); Castro de Abreu, Isabel Maria, Dra., Urb. do Infantado, 2670-386 Loures (PT)
(74) Representative: Ferreira, Maria Silvina

(56) References cited:
- WO-A-02/04004
- WO-A-2005/018530
- DATABASE WPI Section Ch, Week 200429 Derwent Publications Ltd., London, GB; Class B05, AN 2004-305342 XP002364504 & CN 1 470 236 A (WUHAN NO 4 HOSPITAL) 28 January 2004 (2004-01-28)
- WOLF B ET AL: "UNTERSUCHUNGEN ZUR KOMPATIBILITAET VON WIRKSTOFFHALTIGEN DERMATIKA MIT ZUSAETZLICH EINGEARBEITETEN WIRKSTOFFEN" PHARMAZIE, DIE, GOVI VERLAG, ESCHBORN, DE, vol. 51, no. 12, 1996, pages 966-970, XP000651797 ISSN: 0031-7144

## Description

### Field of the invention

The present invention relates to a pharmaceutical composition, which combines a substance with anti-oxidant activity, ubidecarenone, with a healing inducing substance, dexpanthenol, and with a substance with antiseptic and disinfectant activity, chlorhexidine or a salt thereof, as well as a method to prepare the pharmaceutical composition which includes the said association and to the respective use for the treatment of anal diseases, examples of which are anal fissures (acute, chronic and secondary to other clinical conditions).

### Description of the Prior Art

The anal canal is, functionally, an area, which extends from the ano-rectal ring to the edge of the anal orifice. The upper portion is lined, mainly, with cylindrical epithelium, while the area distal to the dented line is lined with a squamous stratified epithelium, without follicular or sweat glands (anodermis).

In developed societies, the diseases that affect the anus and/or the rectum present increasing rates of prevalence and incidence. Amongst the diseases that affect the anus and the rectum are prominent in terms of public health, hemorrhoids (internal an external) and anal fissures (acute anal fissure, chronic anal fissure and anal fissure secondary to the inflammatory bowel disease, cancers and infectious processes).

Anal fissure is an ulcer in the anodermis, with longitudinal orientation, which extends from the edge of the anus to the mucocutaneous junction (dented line), located preferably in the rear middle line (90-98%). The majority of these fissures heal spontaneously. However, a small proportion of these lesions does not heal and evolves into chronicity (traditionally defined as the presence of symptoms longer than 6 weeks of evolution or, when there is pain with less duration, in the prior history other similar occurrences are referred to). Therapy for acute anal fissure is conservative, based on recommendation of local and life hygiene habits (e.g.: diet with fibres and regular physical activity), as well as the application of topical preparations containing various combinations of analgesics (lidocaine, tetracaine, cinchoine or tetracaine) anti-inflammatories (hydrocortisone, prednisolone, fluocortone), antiseptics (hexachlorophene, ethyl p-aminobenzoate), descongestants (phenylephrine, calcium dobesilate). Administration of topical preparations containing non-steroidal anti-inflammatories drugs (NSAID's), corticosteroids or immunossupressors induce the inhibition of prostaglandins synthesis, DNA synthesis and cell replication, cell migration (ex: keratinocytes) that can lead to the delay of the reparation process (reepithelization, regeneration and healing), as well as the atrophy of adjacent anodermis.

Accordingly, a method for treating acute anal fissure should include the application of substances whose mechanisms of action go against the underlying physiopathological conditions, namely an active substance with potent anti-oxidant activity such as ubidecarenone, associated to a substance with inducing effect of the mucosal reparation process, specifically of the epithelium (reepithelization), as it is the case of dexpanthenol, and a substance with antiseptic and disinfectant activity.

Ubidecarenone, also designated Coenzyme Q₁₀ (because it presents a lateral chain formed by 10 isoprenic units) is a lipossoluble quinolone, which takes part in the electron transfer in the mitochondrial respiratory chain. Reduced ubidecarenone presents indol groups once it accepts hydrogens, but it is capable of oxidising and creating a reducing environment when giving these very hydrogens. Ubidecarenone presents cytoprotector effect of the squamous epithelium cells and anodermis fibroblasts. This cytoprotector action originates from the inhibition of lipid peroxidation (stabilisation of cell membranes) and from the prevention of occurrence lesions of the cell desoxirribonucleic acid (DNA). Thus, ubidecarenone presents a very important factor of control of cell death by necrosis and by apoptosis. (Martindale. The Complete Drug Reference, 33rd edition, The Pharmaceutical Press). Topical application of ubidecarenone has a strong anti-erythematogenous effect (Cosmetic Science).

Chlorhexidine is an antiseptic and disinfectant of broad spectrum, while dexpanthenol (pantotenic acid) presents essentially a regenerating effect of the injured mucous membrane.

Dexpanthenol is a stable alcoholic precursor of the pantotenic acid, which represents the biologically active substance. In the tissues, including the skin, dexpanthenol is rapid and completely metabolised into pantotenic acid. Pantotenic acid is involved in the synthesis of coenzyme A, playing an important role in the carbohydrates, fatty acids and proteins metabolism, gliconeogenesis, synthesis of steroid hormones and porfirines (Effect of Topically Applied Dexpanthenol on Epidermal Barrier Function and Stratum Corneum Hydration. Arzneim.-Forsch./Drug Res 50 (II), 659-663 [2000]; Topical use of dexpanthenol in skin disorders. Am. J. Clin. Dermatol. 3 (6): 427-433 [2002]). Dexpanthenol acts as a humectant, improving *stratum corneum* hydration, reducing transdermal loss of water and promoting skin elasticity. On the other hand, its cutaneous regeneration activity results from the activation of fibroblasts proliferation, which is relevant in the healing of wounds. Dexpanthenol further aids the accelerated reepithelization of the wound such as it is monitored by the reduction of loss of water via transdermal and decreases the itching sensation. It presents a reduced risk of irritation or skin sensitivity (Martindale. The Complete Drug Reference, 33rd edition, The Pharmaceutical Press).

Chlorhexidine is a cationic bis-biguanide, bactericide or bacteriostatic against a wide spectrum of bacteria (Gram-negatives and Gram-positives), fungicide and virucide. It presents a low toxicity in mammals. Its mechanism of action is the disruption of the cell membrane, causing growth inhibition dependant on concentration, and cell death. It has been used in various clinical conditions and situations such as pre-surgery disinfecting of the skin, wounds and burns surfaces, for washing hands and in the oral cavity, in reducing the accumulation of dental plaque in the prevention of occurrence of complication after performance of dental procedures or in mouth sores, as well as in the reincidence of these lesions (The Pharmaceutical Codex, 11th edition; The Complete Druq Reference. Martindale. 33rd edition. Pharmaceutical Press). Its use is not associated with the increase in the incidence of antibiotics resistance. Its application on open wounds does not delay the healing process, such as happens, for example, with polyvinilpirrolidone.

Chronic anal fissure characterises by the presence of an ulcer with a hard and fibrous margin, by the possibility of observation of fibres of the internal anal sphincter in the bottom of the ulcer and by the occurrence of a skin wrinkle in the distal end or a hypertrofic papillae in the proximal margin.

In the pathogenesis of chronic anal fissure it is accepted that the pathological process starts with a local trauma from which results a wound, a spasm of de internal anal sphincter and a reduction in the blood flow in the anal mucous membrane. These two latter factors concur for the start of a persisting ulcer. The ischemic theory supports the use of new treatments that aim at the reduction of pressure in the internal anal sphincter at rest and thereby improve blood flow in the rear anal commissure.

There are three therapeutic options in the chronic anal fissure: surgical, topical and the application of botulinic toxin, each one presenting advantages and disadvantages (McCallion K, Progress in the understanding and treatment of chronic anal fissure. Postgraduate Medical Journal 2001; 77: 753-758). The surgical therapeutics of the chronic anal fissure are the internal lateral sphincteretomy and anal dilatation. Both techniques may cause significant morbidity, namely as sphincter incontinence (30%). Therefore, a non-surgical method is preferred. The disfunction of the internal anal sphincter contractibility can also be modulated by the topical or oral administration of compounds with myorelaxing activity or with the application of botulinic toxin.

As aforementioned, anal trauma (eventually caused by the continuing passage of hard faeces), particularly if associated to local conditions of deficiency of arterial irrigation of the mucous membrane and muscular layer is one of the underlying mechanisms for the installation of anal fissure. On the other hand, the inflammatory process around the fissure settles rapidly and may evolve into chronicity. Finally, anus and rectum present a rich bacterial flora, which contributes for the persistency of the inflammatory process.

Therefore, the therapeutics of chronic anal fissure should benefit from the administration of ubidecarenone as a potent anti-oxidant, of dexpanthenol as an inductor of the healing process of the anal mucous membrane and, finally, of chlorhexidine a local, potent and wide spectrum antiseptic and disinfectant. Besides the increased therapeutic benefit, an increased acceptance from the patient to the instituted therapy is expected.

However, it is part of the pharmaceutical knowledge that, with reference to quality requirements, there is a great difficulty in preparing a pharmaceutical formulation containing three different active substances, not only due to the physiochemical incompatibilities between the different active substances, but also between each active substance and various excipients with different properties, and also between the excipients themselves. As it is known to one of skill in the art, the stability of substances is less when carried in the liquid state rather than in the solid state.

When substances are formulated, dissolved in a suitable carrier, they are subject to hydrolyse and oxidation phenomena, which provoke degradation of these substances. (Carstensem, J. T. & Musa, M.N. (1972) J. Pharm. Sci 61 1112.; Higuchi, T., Marcus, A. D. & Bias, C. D. (1954) J. Am. Pharm. Assoc. Sci. 60 1145); Chemical stability of pharmaceuticals (1979) Connors, K. A., Amidon, G. L. & Kennon, L. Wiley, New York; Kinetics and Mechanisms in stability of drugs (1967) Garrett, E. R. In: H. S. Bean, A. H. Beckett & J. E. Carless, Advances in Pharmaceutical Sciences, vol. 2, Academic Press, New York).

The formulation of semi-solid pharmaceutical forms becomes very complicated due to the various components used and possible chemical interactions, which can be of active-excipient, excipient-excipient and active-active nature.

In the prior art there are disclosed some associations which involve ubidecarenone, dexpanthenol or chlorhexidine, being, however, novel the association of these three pharmacologically active substances, namely:
- Patent DE20215776 relates to a cosmetic preparation for use in piercing, containing dexpanthenol, chlorhexidine gluconate and glycerol;
- Patent DE3720048 relates to a healing gel containing sulphonamide and dexpanthenol for use in decubitus ulcers;
- Patent EP787005 relates to an association of dexpanthenol with benzoyl peroxide for treating skin lesions;
- Patent US5985291 relates to the treatment of topical bacterial infections with chlorhexidine and mupirocin;
- Patent WO01/19330 A1 relates to compositions, which contain dexpanthenol and glycine useful for treating dermatological disorders that involve the degranulation of mastocytes.

Compositions containing ubidecarenone, dexpanthenol or chlorhexidine are also known, namely:
- Patent US4654373 discloses the use of coenzyme Q₁₀ for topical application and cutaneous treatment of skin diseases;
- Patent EP146742 relates to compositions containing Ubidecarenone useful for treating ulcers caused by radiotherapy;
- Patent EP0261362 relates to a formulation containing ubidecarenone for application to wounds;
- Patent EP0208833 relates to the treatment of decubitus ulcers by the application of a cream, lotion or ointment containing ubidecarenone;
- Patent US2003/0105168 A1 discloses compositions containing ubiquinone;
- Patent EP610655 relates to sterile formulations with dexpanthenol;
- Patent GB1599159 claims pharmaceutical compositions containing chlorhexidine;
- Patent US2004/0191274 A1 relates to a hydroalcoholic gel containing chlorhexidine, useful for disinfecting hands without causing the destruction of the skin natural protection barrier.
- Patent WO0204004 discloses oil-in-water wax dispersions containing ubidecarenone, dexpanthenol and chlorohexidine individually
- Patent WO2005018530 discloses an alcohol-free foam useful for rectal applications.
- Patent CN1470236 discloses a multi-component preparation for the treatment of hemorrhoids and anal fissure containing chorohexidine.
- Wolf et al (Pharmazie 51, (12) 1996. pp 966-969) disclose a compatibility study of dermatological formulations of type W/O and O/W with one or several drugs added.

The preparation of a pharmaceutical formulation for topical administration of various active components with such different solubility characteristics poses various obstacles, namely its solubilization in order to be able to have some capability of penetration in the dermis where it will exhibit pharmacological activity. The pharmacological activity of substances is dependent on its capability to solubilize in the physiological means, penetrating through the complex skin structures and reaching the sites of action.

The modulation of percutaneous penetration is done through the careful selection of excipients and pharmaceutical form. The present invention deals with an oil-in-water (O/W) emulsion in which the substance with anti-radical activity (Coenzyme Q₁₀) is solubilized in the oil phase of the formulation and dispersed in the aqueous phase where is found the pantotenic acid precursor (dexpanthenol) with humectant properties. The substance with antiseptic activity, chlorhexidine, is suspended in a non-ionic surfactant agent which allows for its homogeneous distribution through the phases of the emulsion, allowing an effective activity against the micro-organisms that inhabit the affected areas.

Surprisingly, we found that the association of ubidecarenone with dexpanthenol and chlorhexidine or a pharmaceutically acceptable salt thereof and specific excipients enabled the development of a suitable, stable pharmaceutical composition for cutaneous application in anal diseases, such as acute anal fissure and chronic anal fissure.

### Detailed Description of the Invention

It is an object of the present invention the combination of ubidecarenone with dexpanthenol and with chlorhexidine or a pharmaceutically acceptable salt thereof.

It is an object of the present invention a pharmaceutical composition, which combines ubidecarenone with dexpanthenol and with chlorhexidine or a pharmaceutically acceptable salt thereof and that is simultaneously stable and suitable for cutaneous application.

It is also an object of the present invention the preparation of a pharmaceutical composition, related to the novel triple association formed by ubidecarenone, dexpanthenol and chlorhexidine or a pharmaceutically acceptable salt thereof, simultaneously stable and suitable for cutaneous application.

A fourth object of the present invention is the use of the said pharmaceutical composition, comprising a combination of ubidecarenone, dexpanthenol and chlorhexidine for the preparation of a medicament for the treatment of anal diseases, namely acute anal fissure and chronic anal fissure.

A fifth object of the present invention is the use of the pharmaceutical composition for the preparation of a medicament for treating anal diseases, comprising the combination of ubidecarenone with dexpanthenol and with chlorhexidine or a pharmaceutically acceptable salt thereof, in association with a modulator of the ionic channels.

The composition object of the present invention comprises an association of ubidecarenone, dexpanthenol and chlorhexidine or a pharmaceutically acceptable salt thereof with excipients generally used in formulations for the purpose of cutaneous application.

The composition contains an emollient agent, which consists of a mixture of saturated alcohol esters, between C12 and C18, with fatty acids, namely caprilic acid (octanoic) and capric acid (decanoic). Its concentration varies between 2.0% and 20.0% of the total mass of the composition, preferably between 5.0% and 10.0% of the total mass of the composition.

Emollients are substances which, when incorporated to the skin surface, transmit a very satisfactory sensation of smoothness and flexibility, enhancing the adhesion to the therapy. The majority of skin pathological conditions, where occurs the disturbance of the balance of the lipid mantle of the *stratum corneum*, exhibits an intense desquamation process with unpleasant sensation. The incorporation of an emollient agent reduces the burning sensation, besides aiding in the solubilization of low solubility drugs, such as ubidecarenone.

Due to its ability to melt between the cells of the *stratum corneum*, emollients such as fatty acids esters may, in theory, aid in the enhancement of penetration through the skin of low mobility molecules in the diffusion processes.

There are, in the literature, many examples of agents with skin emollient properties. The selection of the emollient agent can be done taking into consideration the characteristics of the active components and the site of application. Esters of lower molecular weight and with high branching transmit less the sensation of fat than esters of higher molecular weight and less branched.

The composition also contains an oily carrier that consists of a mixture of cyclic hydrocarbons and aliphatic hydrocarbons, between C14 and C18. Ubidecarenone is a highly lipophilic molecule, with high solubility in apolar solvents such as chloroform and dichloromethane. Its optimal concentration varies between 1.0% and 10.0% of the total mass of the composition, preferably between 2.0 and 5.0% of the total mass of the composition.

Preferably, the solubilization of ubidecarenone, using a carrier compatible with the skin, is achieved through the selection of a mixture of apolars hydrocarbons such as liquid paraffin.

Due to its occlusive properties, mixtures of hydrocarbons or mineral oils or paraffins are deposited in the intercell spaces in the skin, forming a protective and insulating layer. The occlusive property is used to make easier the cutaneous penetration of active substances through the decrease of transcell loss of water.

Another constituent of the composition is a thickening agent comprising a mixture of aliphatic alcohols, mainly stearilic alcohol and cetilic alcohol. Preferably, the proportions should be between 50% to 70% of de stearilic alcohol and between 20% to 35% of cetilic alcohol. Still preferably, the total percentage of these two alcohols should be, at least, 90% of the total percentage of fatty alcohols.

Its optimal concentration in the composition varies between 1.0% and 10.0% of the total mass of the composition, preferably between 3.0 and 8.0% of the total mass of the composition.

This excipient, commonly designated ceto-stearilic alcohol, is used in the present invention as an emulsion stabiliser, imparting high physical stability through reduction of coalescence effects in the internal phase. In combination with surfactants, ceto-stearilic alcohol forms emulsions with complex microstructures and, depending on the proportion used, it is possible to obtain lamelar structures, liquid crystals and gels.

On the other hand, the presence of this mixture of fatty alcohols, impart the composition with a capacity of water absorption which can be useful when used with interstitial exsudate production, maintaining the wounds dry and making healing easier.

It is a further constituent of this composition a non-ionic surfactant comprising fatty acids esters with polyethoxilated sorbitol and anhydrides thereof. Preferably, the surfactant should contain 20 mol of ethylene group for each mol of sorbitol. Still preferably, the surfactant should be a palmitic acid monoester. Its optimal concentration in the composition varies between 1.0% and 10.0% of the total mass of the composition, preferably between 2.0 and 5.0% of the total mass of the composition.

The presence of these excipients allows for the adequate homogenization of chlorhexidine in the present invention.

Chlorhexidine, especially its hydrochloride is a substance with very low solubility in various solvents, making difficult its homogenization in the emulsion. On the other hand, chlorhexidine is also incompatible with ionic surfactants, especially anionics.

In relation to substances with pharmacological activity which associates to one another, ubidecarenone is included in the composition, more preferably between 0.5% to 7,5% of the total mass of the composition. Dexpanthenol is included in the composition in concentrations that vary between 1% and 15% of the total mass of the composition, more preferably between 5% to 7.5% of the total mass of the composition. Chlorhexidine or a pharmaceutically acceptable salt thereof is included in the composition in concentrations that vary between 0.1% and 5% of the total mass of the composition, more preferably between 0.1% to 1% of the total mass of the composition.

The oily excipients are selected, from amongst the oily carriers usually used in the semi-solid pharmaceutical forms and mentioned above, those which its association with the three active substances will not disclose incompatibilities and simultaneously will allow the obtention of a formulation with suitable characteristics for cutaneous application.

According to the present invention, the pH of the composition should vary between 5 to 7.5, more preferably between 5.5 to 6.5, and the final viscosity of the composition should be between 100 mPas and 20000 mPas, more preferably between 350 mPas and 5000 mPas, when determined at a temperature between 20 °C and 25 °C.

Following, a form of preparation is summarily described:

### - Preparation of the oily phase

In a container of suitable dimensions, at a temperature between 65 °C to 85 °C, more preferably between 70 °C to 75 °C ceto-stearilic alcohol, liquid paraffin and coco-caprylate/caprate are fused. After the obtention of a homogeneous phase, the temperature is lowered to 50 °C to 60 °C, more preferably to 50 °C and ubidecarenone is added.

### - Preparation of the aqueous phase

In a container of suitable dimensions, part of the water is heated between 70 °C to 80 °C and the preservatives are solubilized. After obtention of a solution, it is allowed to cool to 50 °C. In another container, chlorhexidine or a pharmaceutically acceptable salt thereof is dispersed in polyssorbate 40. After the obtention of a homogeneous dispersion, the contents of the different containers are added, with stirring at 50 °C.

### - Emulsion

The aqueous phase is added to the oily phase with stirring, at 50 °C. It is then allowed to cool very slowly until it reaches room temperature.

Ubidecarenone is a molecule sensitive to temperature, degrading significantly with the rise thereof. The container should be heated, initially at a temperature between 65 °C and 85 °C, so as to liquefy the oily excipients. After liquefaction, ubidecarenone must be solubilized in these oily excipients only, after lowering of the temperature to values between 50 °C and 60 °C, more preferably to 50 °C.

Chlorhexidine is a substance sensitive to temperature. When heated, aqueous solutions of chlorhexidine undergo degradation with the emergence of a related compound, 4-chloroaniline. In the tests carried out with the present composition, chlorhexidine resists, without appreciable degradation, to small passages through high temperatures, at least at 50 °C.

The preparation of emulsion is accomplished, preferably, using the phase inversion method. The aqueous phase is added to the oily phase, slowly and maintaining the temperature of the solution at 50 °C.

The addition phase of the aqueous to the oily phase presented itself very critical for the obtention of a homogeneous emulsion. When the emulsion was rapidly cooled, even maintaining a high velocity of stirring, it was found a rapid solidification of ubidecarenone, forming an orange solid mass suspended in a yellow emulsion, exhibiting a heterogeneous structure. It was found that the way to obtain a stable and homogeneous emulsion was to carry out the addition of phases at 50 °C, cool slowly until 30 °C and maintain vigorous stirring of the emulsion. Afterwards, the solution should be cooled to room temperature, reducing the speed of stirring.

The present invention is illustrated by way of examples, shown below. However, these are not meant to be limiting of the invention.

### EXAMPLE 1: Emulsion of ubidecarenone (7.5%) + dexpanthenol (5.0%) + chlorhexidine (dihydrochloride) (0.5%), m/m

### 1 - Composition

**TABLE 1: QUALITATIVE AND QUANTITATIVE COMPOSITION OF THE EMULSION (BATCH - UCDP21-020172A)**

| Composition | g | % |
|---|---|---|
| ⁽¹⁾Ubidecarenone | 2.25 | 7.50 |
| ⁽²⁾Dexpanthenol | 1.50 | 5.00 |
| ⁽³⁾Chlorhexidine (dihydrochloride) | 0.150 | 0.500 |
| Cetiol LC^{®} (coco-caprylate/caprate) | 3.00 | 10.00 |
| Liquid paraffin | 1.20 | 4.00 |
| Lanette O^{®} (ceto-stearilic alcohol) | 1.80 | 6.00 |
| Tween 40^{®} (polyssorbate 40) | 0.900 | 3.00 |
| Methylparaben (methyl parahydroxibenzoate) | 0.108 | 0.360 |
| Propylparaben (propyl parahydroxibenzoate) | 0.012 | 0.040 |
| ^{(1), (2), (3)}Purified water | 19.08 | 63.60 |
| Mass of 1 tube | 30 g | |

| | | |
|---|---|---|
| ^{(1), (2), (3)} The sum (ubidecarenone + dexpanthenol + chlorhexidine dihydrochloride) + water should be equal to 22,979 g. | | |

### 2 - Preparation method

### - Preparation of the oily phase

In a container of suitable dimensions ceto-stearilic alcohol, liquid paraffin and coco-caprylate/caprate were fused at 70 °C. After the obtention of a homogeneous phase the temperature was reduced to 50 °C and ubidecarenone was added.

### - Preparation of the aqueous phase

In a container of suitable dimensions part of the water was heated at 80 °C and the preservatives were solubilized. After the obtention of a solution, it is allowed to cool to 50 °C. In another container, dexpanthenol was solubilized in part of the water. In another container, chlorhexidine dihydrochloride was dispersed in polyssorbate 40. After the obtention of a homogeneous dispersion, the contents of the different containers were added, with stirring at 50 °C.

### - Emulsion

The aqueous phase was added to the oily phase with stirring, at 50 °C. The temperature was gradually lowered until reaching room temperature.

The emulsion was packed in aluminium tubes with fenolic lining.

### 3 - Evaluation of emulsion stability

The tubes containing the emulsion were stored at 25 °C/60% RH and 60 °C. The samples were analysed at 0 days, 7 days and 21 days for stability. In each period of analysis the formulation was evaluated for the appearance, pH values, assay of the active substances and related compounds. The viscosity of the emulsion was determined (UCDP21-020172A) at 0 days and after 21 days at 25 °C/60% RH and 60 °C. In order to determine this parameter a rotating viscosimeter *(Haake VT550,* with rotor SV1) was used. 50 determinations were carried out (T = 25 °C ± 1°C), at a speed of 200 s⁻¹, during 300 s.

Tables 2 to 12 show the results obtained during the accelerated stability study.

### Appearance

**TABLE 2: APPEARANCE OF EMULSION OF UBIDECARENONE, DEXPANTHENOL AND CHLORHEXIDINE DIHYDROCHLORIDE**

| Time (days) | Storage conditions | |
|---|---|---|
| | 25 °C/60% RH | 60 °C |
| 0 | Brilliant cream of yellow orange colour | |
| 7 | Brilliant cream of yellow orange colour | |
| 21 | Brilliant cream of yellow orange colour | Brilliant cream of yellow orange colour with a slight darkening |

In Table 2 it is possible to observe the description of the appearance of the stored emulsion at 25 °C/60% RH and 60 °C.
**pH**

**TABLE 3: pH VARIATION OF EMULSION OF UBIDECARENONE, DEXPANTHENOL AND CHLORHEXIDINE DIHYDROCHLORIDE**

| Time (days) | Storage conditions | |
|---|---|---|
| | 25 °C/60% RH | 60 °C |
| 0 | 6.05 | -- |
| 7 | 6.08 | 6.02 |
| 21 | 6.04 | 6.01 |

The pH of the ubidecarenone, dexpanthenol and chlorhexidine dihydrochloride emulsion dosed at 7,5%+5,0%+0,5% (m/m) was kept unaltered during the accelerated stability study, even after 21 days at 60 °C.

### Assay of active substances

**TABLE 4: ASSAY (%) OF UBIDECARENONE DURING THE ACCELERATED STABILITY STUDY**

| Time (days) | Storage conditions | |
|---|---|---|
| | 25 °C/60% RH | 60 °C |
| 0 | 102.49 ± 0.89 | -- |
| 7 | 100.01 ± 0.14 | 96.44 ± 0.15 |
| 21 | 99.53 ± 0.17 | 91.00 ± 0.09 |

**TABLE 5: ASSAY (%) OF DEXPANTHENOL DURING THE ACCELERATED STABILITY STUDY**

| Time (days) | Storage conditions | |
|---|---|---|
| | 25 °C/60% RH | 60 °C |
| 0 | 99.60 ± 0.17 | -- |
| 7 | 101.34 ± 0.03 | 99.77 ± 0.23 |
| 21 | 102.05 ± 0.87 | 96.98 ± 0.19 |

**TABLE 6: ASSAY (%) OF CHLORHEXIDINE DIHYDROCHLORIDE DURING THE ACCELERATED STABILITY STUDY**

| Time (days) | Storage conditions | |
|---|---|---|
| | 25 °C/60% RH | 60 °C |
| 0 | 102.44 ± 0.37 | -- |
| 7 | 102.40 ± 0.24 | 91.75 ± 1.86 |
| 21 | 102.92 ± 0.89 | 68.53 ± 0.98 |

### Assay of preservatives

**TABLE 7: ASSAY (%) OF THE PRESERVATIVE METHYLPARABEN DURING THE ACCELERATED STABILITY STUDY**

| Time (days) | Storage conditions | |
|---|---|---|
| | 25 °C/60% RH | 60 °C |
| 0 | 103.07 ± 0.08 | -- |
| 7 | 99.85 ± 0.04 | 98.91 ± 0.65 |
| 21 | 99.11 ± 0.75 | 96.86 ± 0.30 |

**TABLE 8: ASSAY (%) OF THE PRESERVATIVE PROPYLPARABEN DURING THE ACCELERATED STABILITY STUDY**

| Time (days) | Storage conditions | |
|---|---|---|
| | 25 °C/60% RH | 60 °C |
| 0 | 104.57 ± 0.62 | -- |
| 7 | 104.21 ± 0.38 | 102.47 ± 0.17 |
| 21 | 97.94 ± 0.86 | 94.26 ± 0.07 |

### Assay of related compounds

**TABLE 9: ASSAY (%) OF THE RELATED COMPOUNDS OF UBIDECARENONE DURING THE ACCELERATED STABILITY STUDY**

| Time (days) | Storage conditions | |
|---|---|---|
| | 25 °C/60% RH | 60 °C |
| 0 | trr 0.71 (CoQ) = | |
| | 0.329 | -- |
| | Total = 0.329 | |
| 7 | trr 0.71 (CoQ₉) = | trr 0.58 = 0.243 |
| | 0.326 | trr 0.71 (CoQ₉) = |
| | trr 1.44 = 0.021 | 0.315 |
| | | trr 0.76 = 0.357 |
| | | trr 1.44 = 0.042 |
| | Total = 0.347 | Total = 0.957 |
| 21 | trr 0.71 (CoQ₉) = | trr 0.57 = 0.446 |
| | 0.319 | trr 0.71 (CoQ₉) = 0.237 |
| | | trr 0.76 = 1.823 |
| | | trr 0.83 = 0.019 |
| | | |
| | Total = 0.319 | Total = 2.525 |

| | | |
|---|---|---|
| Q.L. (quantification limit) = 0.24% | | |

**TABLE 10: ASSAY (%) OF THE RELATED COMPOUNDS OF DEXPANTHENOL DURING THE ACCELERATED STABILITY STUDY**

| Time (days) | Storage conditions | |
|---|---|---|
| | 25 °C/60% RH | 60 °C |
| 0 | -- | -- |
| 7 | | trr 1.34 = 0.619 |
| | trr 2.07 = 0.599 | |
| | | trr 2.08 = 0.376 |
| | Total = 0.599 | |
| | | Total = 0.996 |
| 21 | trr 1.33 = 0.117 | trr 1.32 = 1.740 |
| | trr 2.04 = 0.514 | trr 2.04 = 0.571 |
| | Total = 0.632 | Total = 2.310 |

| | | |
|---|---|---|
| Q.L. = 0.28% | | |

**TABLE 11: ASSAY (%) OF THE RELATED COMPOUNDS OF CHLORHEXIDINE DIHYDROCHLORIDE DURING THE ACCELERATED STABILITY STUDY**

| Time (days) | Storage conditions | |
|---|---|---|
| | 25 °C/60% RH | 60 °C |
| 0 | trr 0.82 (Imp B) = | |
| | 0.032 | |
| | trr 1.62 = 0.118 | -- |
| | trr 1.94 = 0.125 | |
| | Total = 0.276 | |
| 7 | trr 0.82 (Imp B) = | trr 0.76 (Imp B) = |
| | 0.026 | 0.157 |
| | trr 1.62 = 0.164 | trr 1.63 = 0.228 |
| | trr 1.93 = 0.100 | trr 1.93 = 0.268 |
| | Total = 0.289 | Total = 0.654 |
| 21 | | trr 0.74 (Imp B) = |
| | trr 0.81 (Imp B) = | |
| | | 0.481 |
| | 0.045 | |
| | | trr 1.21 = 0.047 |
| | trr 1.57 = 0.205 | |
| | | trr 1.46 = 0.047 |
| | trr 1.95 = 0.114 | |
| | | trr 1.58 = 0.271 |
| | | trr 1.95 = 0.452 |
| | Total = 0.364 | |
| | | Total = 1.298 |

| | | |
|---|---|---|
| Q.L. = 0.473% | | |

### Viscosity

**TABLE 12: VISCOSITY OF EMULSION (mPAS)**

| Time (days) | Storage conditions | |
|---|---|---|
| | 25 °C/60% RH | 60 °C |
| 0 | 623.12⁽¹⁾ | -- |
| 21 | 621.38⁽¹⁾ | 453.38⁽¹⁾ |

| | | |
|---|---|---|
| (1) - Average of 38 determinations | | |

The preliminary stability study of the active substances and preservatives in the developed formulation, carried out in stress conditions, indicated that the developed formulation would be stable.

It was found an accentuated reduction in the contents of chlorhexidine dihydrochloride, at 60 °C, after 21 days. This fact is due to the sensitivity of this substance to high temperatures (Martindale. The Complete Drug Reference, 32nd edition, The Pharmaceutical Press).

The indication of stability was confirmed with a study of stability in real time (Example 2).

### EXAMPLE 2: Stability evaluation of an emulsion of ubidecarenone (7,5%) + dexpanthenol (5,0%) + chlorhexidine (dihydrochloride) (0,5%), m/m.

The composition and method of preparation are identical to those presented in Example 1. This example corresponds to the batch numbers UCD6-01007-2B, UCD6-01007-2C and UCD6-01007-2D.

### 1- Evaluation of cream stability

The tubes containing cream were stored at 25 °C/60% RH, 30 °C/60% RH and 40 °C/75 RH. The cream was analysed at 0 days, 3 months and 6 months under the three storage conditions and, additionally, at 12 and 24 months for the condition of 25°C/60%RH. The tests carried out consisted in the evaluation of the appearance, determination of pH, assay of the active substances and related compounds.

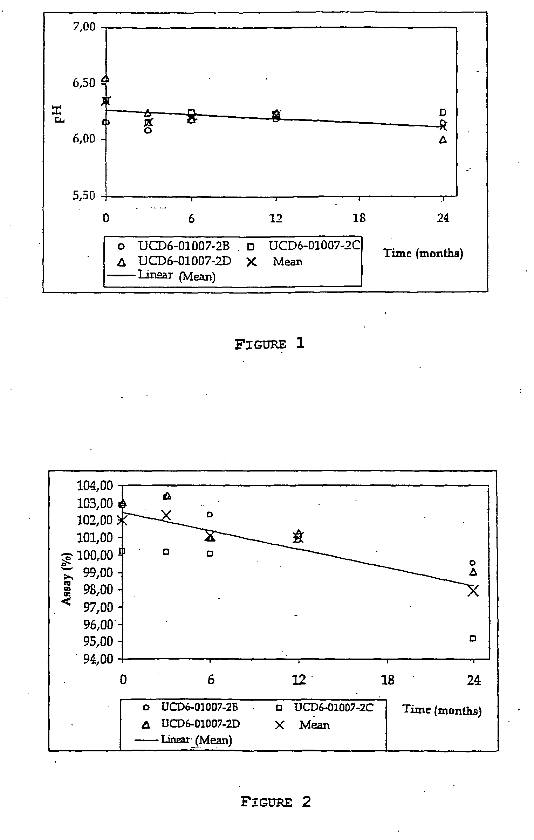

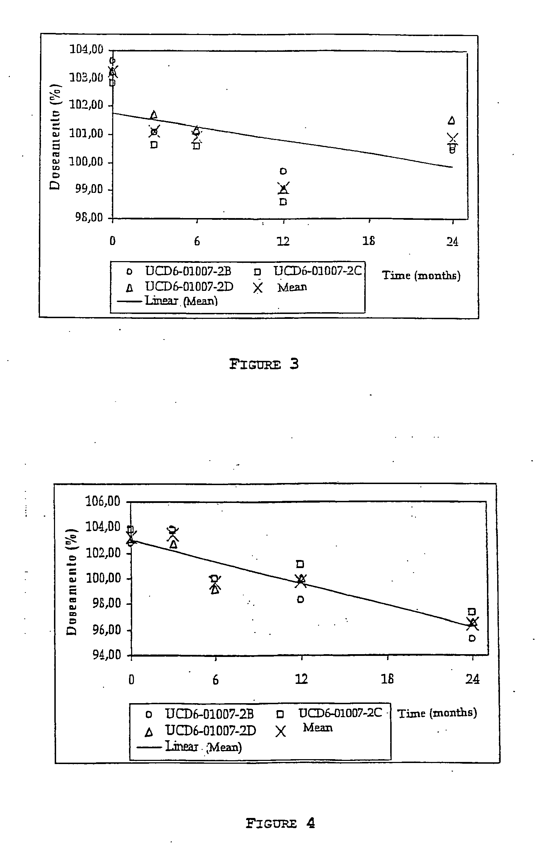

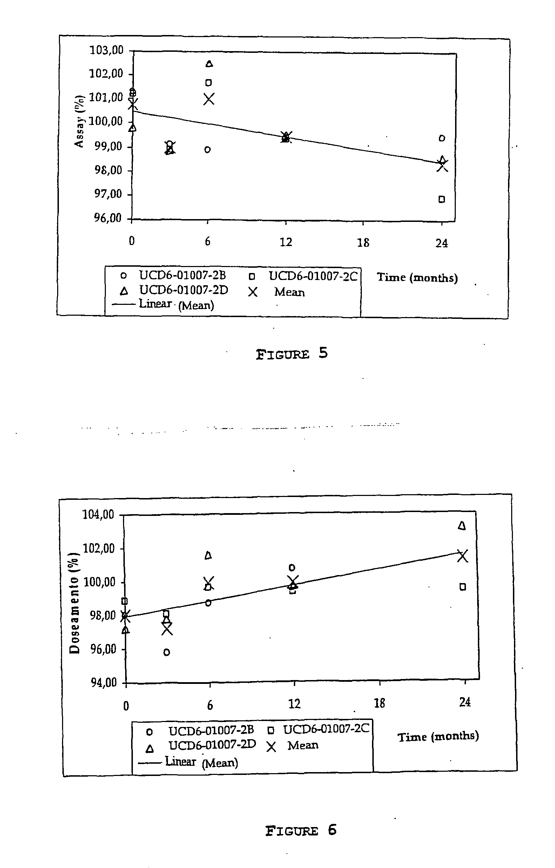

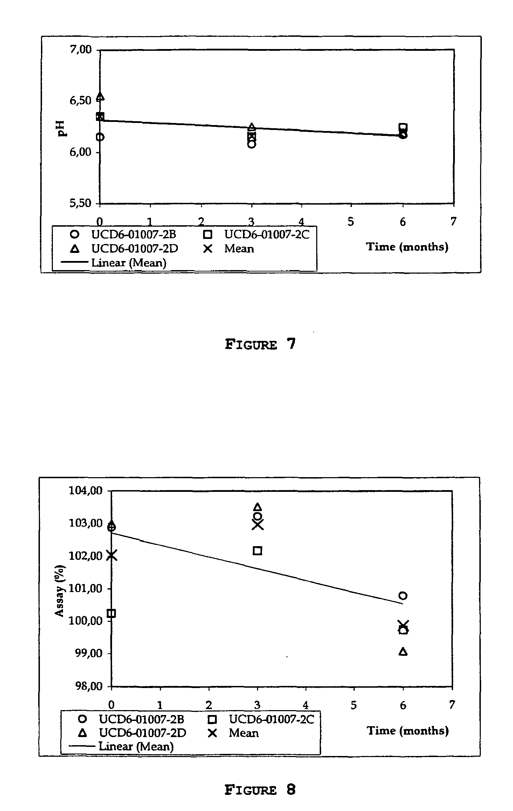

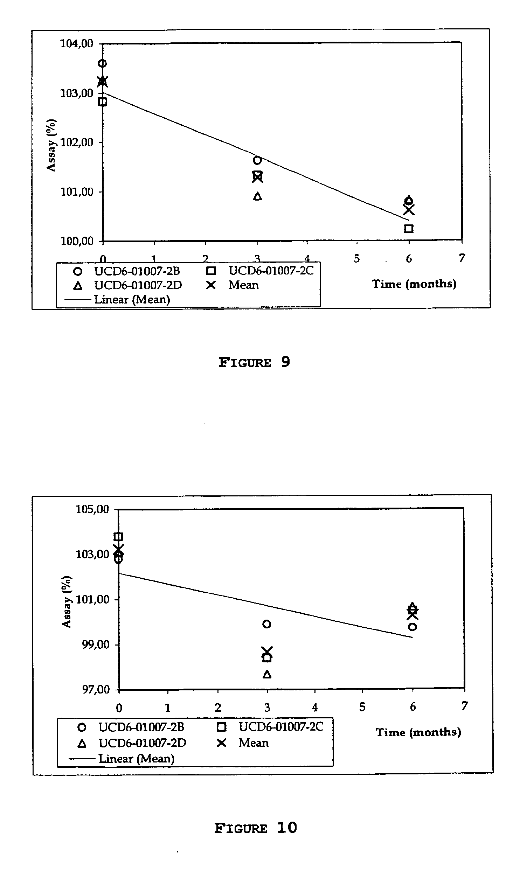

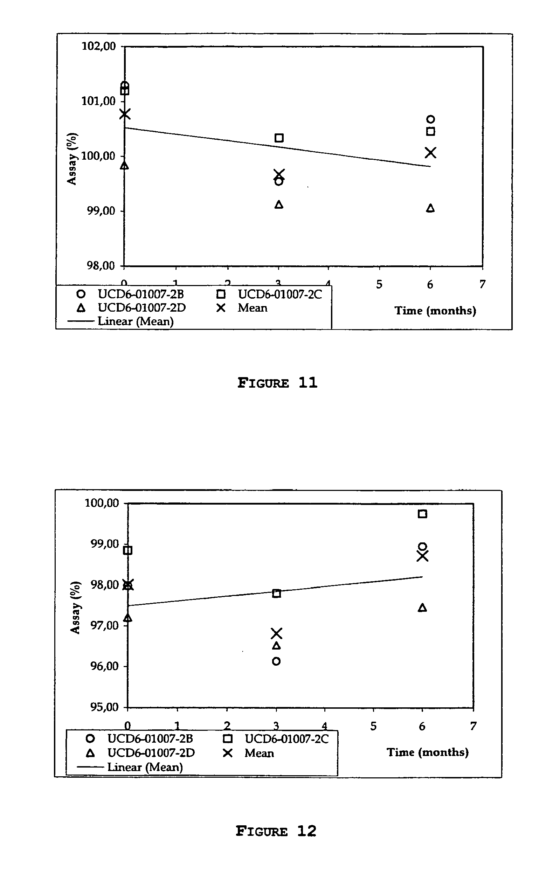

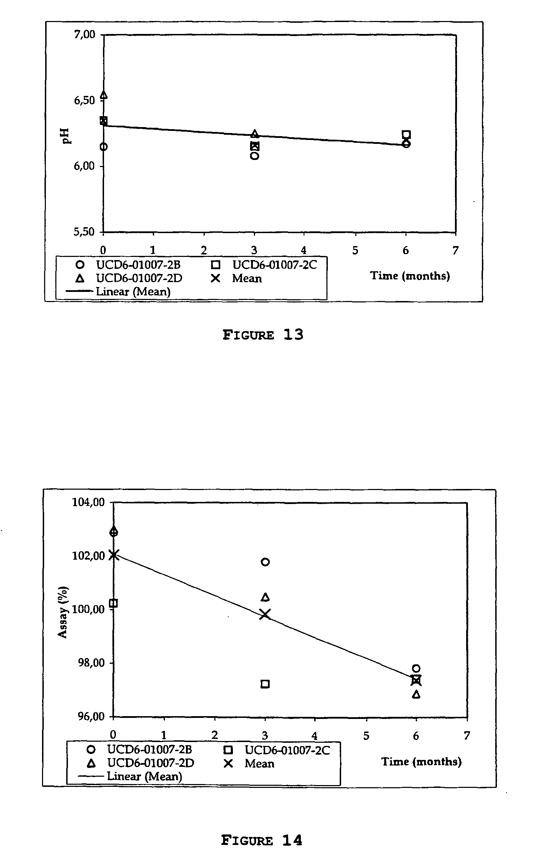

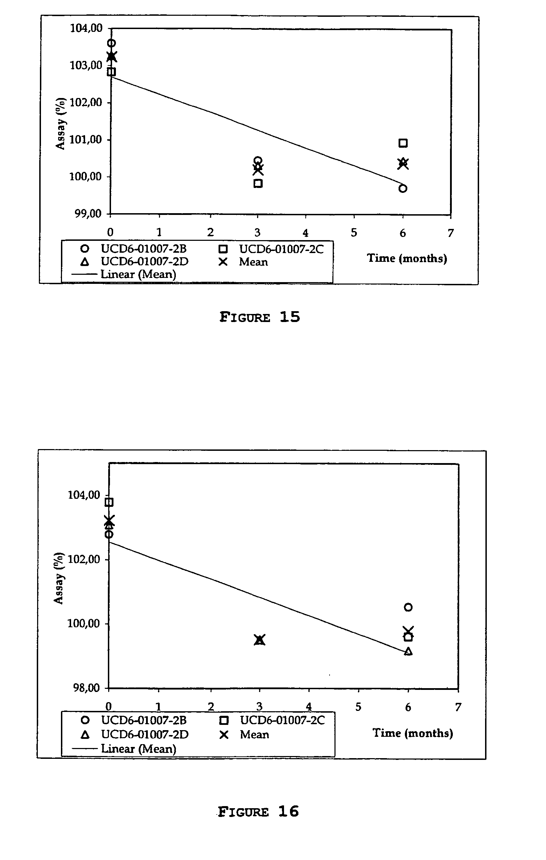

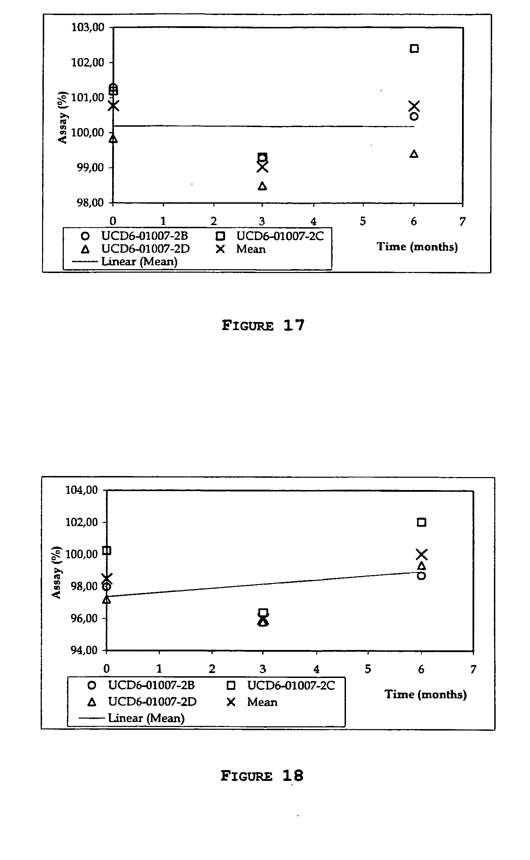

Figures 1 to 18 show the stability study results (pH, assay of the active substances and related compounds) carried out for the cream of ubidecarenone + dexpanthenol + chlorhexidine (dihydrochloride), according to Example 2.

Tables 13 to 21 show in brief the results obtained for the assay of related compounds of the active substances under study.
FIGURE 1: Variation of pH during the stability (24 months, 25 °C/60% RH);
FIGURE 2: Variation of the assay of ubidecarenone during the stability (24 months, 25 °C/60% RH);
FIGURE 3: Variation of the assay of dexpanthenol during the stability (24 months, 25 °C/60% RH);
FIGURE 4: Variation of the assay of chlorhexidine dihydrochloride during the stability (24 months, 25 °C/60% RH);
FIGURE 5: Variation of the assay of methyl p-hydroxybenzoate (24 months, 25 °C/60% RH);
FIGURE 6: Variation of the assay of propyl p-hydroxybenzoate (24 months, 25 °C/60% RH);
FIGURE 7: Variation of pH during the stability (6 months, 30 °C/60% RH);
FIGURE 8: Variation of the assay of ubidecarenone during the stability (6 months, 30 °C/60% RH);
FIGURE 9: Variation of the assay of dexpanthenol during the stability (6 months, 30 °C/60% RH);
FIGURE 10: Variation of the assay of chlorhexidine dihydrochloride during the stability (6 months, 30 °C/60% RH);
FIGURE 11: Variation of the assay of methyl p-hydroxybenzoate (6 months, 30 °C/60% RH);
FIGURE 12: Variation of the assay of propyl p-hydroxybenzoate (6 months, 30 °C/60% RH);
FIGURE 13: Variation of pH during the stability (6 months, 40 °C/75% RH);
FIGURE 14: Variation of the assay of ubidecarenone during the stability (6 months, 40 °C/75% RH);
FIGURE 15: Variation of the assay of dexpanthenol during the stability (6 months, 40°C/75% RH);
FIGURE 16: Variation of the assay of chlorhexidine dihydrochloride during the stability (6 months, 40 °C/75% RH);
FIGURE 17: Variation of the assay of methyl p-hydroxybenzoate (6 months, 40 °C/75% RH;
FIGURE 18: Variation of the assay of propyl p-hydroxybenzoate (6 months, 40 °C/75% RH).

The appearance of the cream was kept unaltered during the period of storage.

The batch UCD6-01007-2B was characterised according to the granulometric distribution (*Coulter LS 130*, optical model *Fraunhofer*, solvent water). The values obtained in terms of particle size were 10% < 0,566 µm; 25% < 1,479 µm; 50% < 4,311 µm, 75%< 8,833 µm; 90%< 12,920 µm.

As it can be seen (Figure 1 to Figure 18), the pharmaceutical composition developed for cutaneous application, presents a high stability, although it has in its composition three active substances of diverse physiochemical characteristics.

The assay value of the three active substances which take part in the composition of the formulation developed, decreased slightly, but never went below 95%, for any of the active substances in the three batches studied (UCD6-01007-2B, UCD6-01007-2C and UCD6-01007-2D). Simultaneously with the decrease of the assay value it was found a slight increase in the contents of related compounds (Table 13 to Table 21). This increase, however, was always very low.

**TABLE 13: LONG TERM STABILITY BATCH UCD6-01007-2B (25 °C ± 2 °C / 60% ± 5% R.H.)**

| **Tests** | | **Start** | **3 months** | **6 months** | **12 months** | **24 months** |
|---|---|---|---|---|---|---|
| Related compounds (%): | | | | | | |
| | | | | | | |
| Ubidecarenone | | | | | | |
| Individuals (%) | | trr 0,68=0,340 | trr 0,68 = | trr 0,69 = | trr 0,70=0,29 | trr 0,70=0,294 |
| | | | 0,398 | 0,277 | trr 1,44=0,06 | trr 0,77=0,175 |
| | Total (%) | 0,340 | 0,348 | 0,277 | 0,35 | 0,469 |
| | | | | | | |
| Dexpanthenol | | | | | | |
| | Individuals (%) | trr 0,58 = | trr 1,20 = | trr 2,04 = | trr 0,82=0,14 | trr 0,51=0,367 |
| | | 0,885 | 0,324 | 0, 541 | trr 1,32=0,33 | trr 0,82=0,202 |
| | | | trr 1,28 = | | | |
| | | | 0,342 | | | |
| | Total (%) | 0,885 | 0,666 | 0,541 | 0,47 | 0,568 |
| | | | | | | |
| ¹Chlorhexidine (dihydrochloride) | | | | | | |
| | Individuals (%) | n.q. | n.q. | n.q. | n.q. | n.q. |
| | | | | | | |
| | Total (%) | -- | -- | -- | -- | -- |

| | | | | | | |
|---|---|---|---|---|---|---|
| ¹Q.L. = 0.473%; n.q. = Non-quantifiable | | | | | | |

**TABLE 14: INTERMEDIATE STABILITY BATCH UCD6-01007-2B (30 °C 2 °C / 60% ± 5% H.R.)**

| **Tests** | | **Start** | **3 months** | **6 months** |
|---|---|---|---|---|
| Related compounds (%): | | | | |
| | | | | |
| Ubidecarenone | | | | |
| | Individuals (%) | trr 0.68 = 0.340 | trr 0.68 = 0.314 | trr 0.69 = 0.234 |
| | Total (%) | 0.340 | 0.314 | 0.234 |
| | | | | |
| Dexpanthenol | | | | |
| | Individuals (%) | trr 0.58 = 0.885 | trr 1.20 = 0.779 | trr 2.04 = 0.575 |
| | | | trr 1.28 = 0.395 | |
| | Total (%) | 0.885 | 1.174 | 0.575 |
| | | | | |
| Chlorhexidine (dihydrochloride) | | | | |
| | Individuals (%) | n.q. | n.q. | n.q. |
| | | | | |
| | Total (%) | -- | -- | -- |

| | | | | |
|---|---|---|---|---|
| ¹Q.L. = 0.473%; n.q. = Non-quantifiable | | | | |

**TABLE 15: ACCELERATED STABILITY BATCH UCD6-01007-2B (40°C 2 °C / 75% ± 5% H.R.)**

| **Tests** | | **Start** | **3 months** | **6 months** |
|---|---|---|---|---|
| Related compounds (%): | | | | |
| | | | | |
| Ubidecarenone | | | | |
| | Individuals (%) | trr 0.68 = 0.340 | trr 0.68 = 0.333 | trr 0.69 = 0.148 |
| | | | trr 0.75 = 0.379 | trr 0.76 = 0.948 |
| | Total (%) | 0.340 | 0.712 | 1.096 |
| | | | | |
| Dexpanthenol | | | | |
| | Individuals (%) | trr 0.58 = 0.885 | trr 1.19 = 0.481 | trr 2.04 = 0.611 |
| | | | trr 1.28 = 0.727 | |
| | Total (%) | 0.885 | 1.208 | 0.611 |
| | | | | |
| ¹Chlorhexidine (dihydrochloride) | | | | |
| | Individuals (%) | n.q. | n.q. | n.q. |
| | | | | |
| | Total 1 (%) | -- | -- | -- |

| | | | | |
|---|---|---|---|---|
| Q.L. = 0.473%. n.q. = Non-quantifiable | | | | |

**TABLE 16: LONG TERM STABILITY BATCH UCD6-01007-2C (25 °C ± 2 °C / 60% ± 5% H.R.)**

| **Tests** | | **Start** | **3 months** | **6 months** | **12 months** | **24 months** |
|---|---|---|---|---|---|---|
| Related compounds (%): | | | | | | |
| | | | | | | |
| Ubidecarenone | | | | | | |
| | Individuals (%) | trr 0,68=0,340 | trr 0,68 = | trr 0,69 = | trr 0,58=0,05 | trr 0,70=0,408 |
| | | | 0,350 | 0,178 | trr 0,70=0,29 | trr 0,77=0,261 |
| | | | | | trr 1,44=0,05 | trr 1,45=0,30 |
| | | | | trr 0,76 = | | |
| | | | | 0,172 | | |
| | Total (%) | 0,340 | 0,350 | 0,350 | 0,39 | 0,7 |
| | | | | | | |
| Dexpanthenol | | | | | | |
| | Individuals (%) | trr 0,57 = 0,420 | trr 1,20 = | trr 2,04 = | Trr 0,82=0,13 | Trr 0,51=0,384 |
| | | | 0,531 | 0,566 | Trr 1,32=0,32 | Trr 0,82=0,182 |
| | | | trr 1,28 = | | | |
| | | | 0,350 | | | |
| | Total (%) | 0,420 | 0,881 | 0,566 | 0,45 | 0,565 |
| | | | | | | |
| ¹Chlorhexidine (dihydrochloride) | | | | | | |
| | Individuals (%) | n.q. | n.q. | n.q. | n.q. | n.q. |
| | | | | | | |
| | Total (%) | -- | -- | -- | -- | --- |

| | | | | | | |
|---|---|---|---|---|---|---|
| ¹Q.L. = 0.473%; n.q. = Non-quantifiable | | | | | | |

**TABLE 17: INTERMEDIATE STABILITY BATCH UCD6-01007-2C (30 °C ± 2 °C / 60% ± 5% H.R.)**

| **Tests** | | **Start** | **3 months** | **6 months** |
|---|---|---|---|---|
| Related compounds (%): | | | | |
| | | | | |
| Ubidecarenone | | | | |
| | Individuals (%) | trr 0.68 = 0.340 | trr 0.68 = 0.350 | trr 0.69 = 0.175 |
| | | | | trr 0.76 = 0.104 |
| | Total (%) | 0.340 | 0.350 | 0.279 |
| | | | | |
| Dexpanthenol | | | | |
| | | | | |
| | Individuals (%) | trr 0.57 = 0.420 | trr 1.28 = 1.109 | trr 2.04 = 0.568 |
| | | | | |
| | Total (%) | 0.420 | 1.109 | 0.568 |
| | | | | |
| ¹Chlorhexidine (dihydrochloride) | | | | |
| | Individuals (%) | n.q. | n.q. | n.q. |
| | | | | |
| | Total (%) | -- | -- | -- |

| | | | | |
|---|---|---|---|---|
| ¹Q.L. = 0.473%; n.q. = Non-quantifiable | | | | |

**TABLE 18: ACCELERATED STABILITY BATCH UCD6-01007-2C (40 °C ± 2 °C / 75% ± 5% H.R. )**

| **Tests** | | **Start** | **3 months** | **6 months** |
|---|---|---|---|---|
| Related compounds (%): | | | | |
| | | | | |
| Ubidecarenone | | | | |
| | Individuals (%) | trr 0.68 = 0.340 | trr 0.68 = 0.340 | trr 0.69 = 0.121 |
| | | | | trr 0.76 = 0.855 |
| | Total (%) | 0.340 | 0.340 | 0.976 |
| | | | | |
| Dexpanthenol | | | | |
| | Individuals (%) | trr 0.57 = 0.420 | trr 0.57 = 0.420 | trr 2.04 = 0.634 |
| | | | | |
| | Total (%) | 0.420 | 0.420 | 0.634 |
| | | | | |
| ¹Chlorhexidine (dihydrochloride) | | | | |
| | Individuals (%) | n.q. | n.q. | trr 0.65 =0.696 |
| | | | | |
| | Total (%) | -- | -- | 0.696 |

| | | | | |
|---|---|---|---|---|
| ¹Q.L. = 0.473%; n.q. = Non-quantifiable | | | | |

**TABLE 19: LONG TERM STABILITY BATCH UCD6-01007-2D (25 °C ± 2 °C / 60% ± 5% H.R.)**

| **Tests** | | **Start** | **3 months** | **6 months** | **12 months** | **24 months** |
|---|---|---|---|---|---|---|
| Related compounds (%): | | | | | | |
| | | | | | | |
| Ubidecarenone | | | | | | |
| | | | | | Trr 0,70=0,29 | Trr 0,87=0,317 |
| | Individuals (%) | trr 0,68 = 0,33 | trr 0,68 = 0,352 | trr 0,69 = 0,240 | Trr 1.,49=0,06 | Trr 0,96=0,209 |
| | | | | | | Trr 1,81=0,030 |
| | | | | | | |
| | Total (%) | 0,33 | 0,352 | 0,240 | 0,35 | 0,556 |
| | | | | | | |
| Dexpanthenol | | | | | | |
| Individuals (%) | | trr 0,84 = | trr 0,57 = 0,420 | trr 2,04 = 0,658 | Trr 0,82=0,12 | Trr 0,51=0,316 |
| | | 0,180 | | | Trr 1,32=0,33 | Trr 0,82=0,165 |
| | | | | | | |
| | Total (%) | 0,180 | 0,420 | 0,658 | 0,45 | 0,481 |
| | | | | | | |
| ¹Chlorhexidine (dihydrochloride) | | | | | | |
| | Individuals (%) | n.q. | n.q. | n.q. | n.q. | n.q. |
| | | | | | | |
| | Total (%) | -- | -- | -- | -- | -- |

| | | | | | | |
|---|---|---|---|---|---|---|
| ¹Q,L. = 0.473%; n.q. = Non-quantifialale | | | | | | |

**TABLE 20: INTERMEDIATE STABILITY BATCH UCD6-01007-2D (30 °C ± 2 °C / 60% ± 5% H.R.)**

| **Tests** | | **Start** | **3 months** | **6 months** |
|---|---|---|---|---|
| Related compounds (%): | | | | |
| | | | | |
| Ubidecarenone | | | | |
| | Individuals (%) | trr 0.68 = 0.33 | trr 0.68 = 0.352 | trr 0.69 = 0.254 |
| | | | | |
| | Total (%) | 0.33 | 0.352 | 0.254 |
| | | | | |
| Dexpanthenol | | | | |
| | Individuals (%) | trr 0.84 = 0.180 | trr 1.29 = 0.926 | trr 2.04 = 0.617 |
| | | | | |
| | Total (%) | 0.180 | 0.926 | 0.617 |
| | | | | |
| ¹Chlorhexidine (dihydrochloride) | | | | |
| | Individuals (%) | n.q. | n.q. | n.q. |
| | | | | |
| | Total (%) | -- | -- | -- |

| | | | | |
|---|---|---|---|---|
| ¹Q.L. = 0.473%; n.q. = Non-quantifiable | | | | |

**TABLE 21: ACCELERATED STABILITY BATCH UCD6-01007-2D (40 °C ± 2 °C / 75% ± 5% H.R.)**

| **Tests** | | **Start** | **3 months** | **6 months** |
|---|---|---|---|---|
| Related compounds (%): | | | | |
| | | | | |
| Ubidecarenone | | | | |
| | Individuals (%) | trr 0.68 = 0.33 | trr 0.68 = 0.339 | trr 0.69 = 0.132 |
| | | | trr 0.75 = 0.393 | trr 0.76 = 0.715 |
| | Total (%) | 0.33 | 0.732 | 0.847 |
| | | | | |
| Dexpanthenol | | | | |
| | Individuals (%) | trr 0.84 = 0.180 | trr 1.28 = 0.781 | trr 2.04 = 0.656 |
| | | | | |
| | Total (%) | 0.180 | 0.781 | 0.656 |
| | | | | |
| ¹Chlorhexidine (dihydrochloride) | | | | |
| | Individuals (%) | n.q. | n.q. | trr 0.65 = 0.703 |
| | | | | |
| | Total (%) | -- | -- | 0.703 |

| | | | | |
|---|---|---|---|---|
| ¹Q.L. = 0.473%; n.q. = Non-quantifiable | | | | |

### EXAMPLE 3: Clinical study

In a clinical test carried out in patients suffering from acute anal fissure, the effectiveness and tolerability of a ubidecarenone, dexpanthenol and chlorhexidine emulsion were evaluated as a treatment method associated to hygienic and dietary measures in general.

The effectiveness evaluations were carried out after 4, 14 and 30 days of treatment. There were considered as evaluation parameters the end of solution of continuity, as well as the evolution of pain, haemorrhage and pruritus through an analogue visual scale.

### EXAMPLE 4: Clinical study

In a comparative clinical study and with random distribution, the emulsion formed by the combination of a ubidecarenone, dexpanthenol and chlorhexidine dihydrochloride was associated, in one of two treatment groups, with a modulator of ionic channels.

There were included patients suffering from anal fissure. An evaluation of the effectiveness was carried out at 2, 4 and 8 weeks of therapy. There were considered as evaluation parameters the end of solution of continuity, as well as the evolution of pain, haemorrhage and pruritus through an analogue visual scale. Lastly, it was considered important to evaluate the rate of reincidence of the anodermis solution after 24 weeks.

## Claims

1. A stable and suitable for cutaneous application combination of ubidecarenone with dexpanthenol and chlorhexidine or a pharmaceutically acceptable salt thereof **characterized in that** it is presented under the form of an oil-in-water emulsion containing ubidecarenone solubilized in the oily phase, chlorhexidine suspended in a non-ionic surfactant agent and dexpanthenol dissolved in the aqueous phase.

2. A pharmaceutical composition stable and suitable for cutaneous application comprising the combination according to claim 1 and topical excipients and/or adjuvants pharmaceutically acceptable.

3. A pharmaceutical composition according to claim 2, **characterised in that** the ubidecarenone is present in a concentration between 0.1% and 15% of the mass of the composition, more preferably between 0.5% to 7.5% of the mass of the composition.

4. A pharmaceutical composition according to claim 2, **characterised in that** the dexpanthenol is present in a concentration between 1% and 15% of the mass of the composition, more preferably between 0.5% to 7.5% of the mass of the composition.

5. A pharmaceutical composition according to claim 2, **characterised in that** the chlorhexidine is present in a concentration between 0.1% and 5% of the mass of the composition, more preferably between 0.1% to 1% of the mass of the composition.

6. A pharmaceutical composition according to claim 2, **characterised in that** the excipients and/or adjuvants pharmaceutically acceptable are selected from one or more emollients, one or more oily carriers, one or more thickening agents, one or more surfactant agents and one or more preservatives.

7. A pharmaceutical composition according to claim 6, **characterised in that** is present as emollient, a mixture of saturated alcohol esters, between C12 and C18, with fatty acids, namely caprilic and capric acid.

8. A pharmaceutical composition according to claim 7, **characterised in that** the emollient is present in a concentration between 2.0% and 20.0% of the mass of the composition, more preferably between 5.0% and 10.0% of the mass of the composition.

9. A pharmaceutical composition according to claim 6, **characterised in that** is present as an oily carrier, a mixture of cyclic hydrocarbons and aliphatic hydrocarbons, between C14 and C18.

10. A pharmaceutical composition according to claim 9, **characterised in that** the oily carrier is present in a concentration between 1.0% and 10.0% of the mass of the composition, more preferably between 2.0% and 5.0% of the mass of the composition.

11. A pharmaceutical composition according to claim 6, **characterised in that** is present as a thickening agent, a mixture of aliphatic alcohols, mainly stearilic alcohol and cetilic alcohol.

12. A pharmaceutical composition according to claim 11, **characterised in that** the thickening agent is present in a concentration between 1.0% and 10.0% of the mass of the composition, more preferably between 3.0% and 8.0% of the mass of the composition.

13. A pharmaceutical composition according to claim 6, **characterised in that** is present as surface tension modifying agents, non-ionic surfactants comprised between fatty acids esters with polyethoxilated sorbitol and/or anhydrides thereof.

14. A pharmaceutical composition according to claim 13, **characterised in that** the surface tension modifying agent is present in a concentration between 1.0% and 10.0% of the mass of the composition, more preferably between 2.0% and 5.0% of the mass of the composition.

15. A pharmaceutical composition according to claim 6, **characterised in that** it contains as preservatives methyl parahydroxibenzoate and/or propyl parahydroxibenzoate or propyleneglycol.

16. A pharmaceutical composition according to any one of claims 2 to 15, **characterised in that** it is presented under the form of an oil-in-water emulsion (O/W).

17. A pharmaceutical composition according to any one of claims 2 to 16, **characterised in that** it presents a pH that varies between 5 and 7.5, more preferably between 5.5 to 7.5.

18. A pharmaceutical composition according to any one of claims 2 to 17, **characterised in that** it presents a viscosity value which varies between 100 mPas and 20000 mPas, more preferably between 350 mPas to 5000 mPas, at 25 °C.

19. A pharmaceutical composition according to claim 2, **characterised in that** it presents physiochemical stability during a minimal period of 2 years.

20. The process for preparing a stable and suitable for cutaneous application combination of ubidecarenone with dexpanthenol and chlorhexidine or a pharmaceutically acceptable salt thereof according to any one of claims 1 to 19, **characterised in that** it comprises the steps of:
a) preparation of an oily phase to which ubidecarenone is added at a temperature between 50 °C and 60 °C;
b) preparation of an aqueous phase in which chlorhexidine or a pharmaceutically acceptable salt thereof is dispersed in the surfactant agent and dexpanthenol is dissolved in water;
c) emulsion at a temperature between 50 °C and 60 °C.

21. The combination according to claim 1 for use as a medicament.

22. The combination according to claim 1 for use in the treatment of acute anal fissure.

23. The combination according to claim 1 for use in the treatment of chronic anal fissure.

24. A pharmaceutical composition stable and suitable for cutaneous application, according to any one of claims 2 to 19, containing the combination of claim 1, **characterised** for use in the treatment of acute anal fissure.

25. A pharmaceutical composition stable and suitable for cutaneous application, according to any one of claims 2 to 19, containing the combination of claim 1, **characterised** for use in the treatment of chronic anal fissure.

26. The use of the stable and suitable for cutaneous application combination according to claim 1 in the preparation of a stable medicament for the treatment of acute anal fissure.

27. The use of the stable and suitable for cutaneous application combination according to claim 1 in the preparation of a stable medicament for the treatment of chronic anal fissure.

28. The use of the stable and suitable for cutaneous application combination according to claim 1 in the preparation of a stable medicament for the prevention of reincidence of acute anal fissure.

29. The use of the stable and suitable for cutaneous application combination according to claim 1 associated to a modulator of the ionic channels, in the preparation of a medicament for the treatment of chronic anal fissure.

30. The use of the stable and suitable for cutaneous application combination according to claim 1 associated to a modulator of the ionic channels, in the preparation of a medicament for the prevention of reincidence of chronic anal fissure.

## Patentansprüche

1. Eine stabile und für Hautanwendung geeignete Zubereitung aus Ubidecarenon mit Dexpanthenol und Chlorhexidin oder einem pharmazeutischen annehmbaren Salz derselben, **dadurch gekennzeichnet, daß** sie als eine Öl-in-Wasser Emulsion vorliegt, welche das Ubidecarenon in der Ölphase gelöst, das Chlorhexidin als einer Suspension in einem nicht-ionischen oberflächenaktiven Mittel und das Dexpanthenol in der wässrigen Phase gelöst, enthält.

2. Eine stabile und für Hautanwendung geeignete pharmazeutische Zusammensetzung, welche die Zubereitung nach Anspruch 1 und pharmazeutische annehmbare topische Hilfsstoffe und/oder Zusatzstoffe umfaßt.

3. Pharmazeutische Zusammensetzung nach Anspruch 2, **dadurch gekennzeichnet, daß** sie das Ubidecarenon in einer Konzentration zwischen 0,1% und 15% der Masse der Zusammensetzung enthält, noch vorzugsweise zwischen 0,5% und 7,5% der Masse der Zusammensetzung enthält.

4. Pharmazeutische Zusammensetzung nach Anspruch 2, **dadurch gekennzeichnet, daß** sie das Dexpanthenol in einer Konzentration zwischen 1% und 15% der Masse der Zusammensetzung enthält, noch vorzugsweise zwischen 0,5% und 7,5% der Masse der Zusammensetzung enthält.

5. Pharmazeutische Zusammensetzung nach Anspruch 2, **dadurch gekennzeichnet, daß** sie das Chlorhexidin in einer Konzentration zwischen 0,1% und 5% der Masse der Zusammensetzung enthält, noch vorzugsweise zwischen 0,1% und 1% der Masse der Zusammensetzung enthält.

6. Pharmazeutische Zusammensetzung nach Anspruch 2, **dadurch gekennzeichnet, daß** die pharmazeutische annehmbare Hilfsstoffe und/oder Zusatzstoffe zwischen einem oder mehr erweichenden Mitteln, einem oder mehr öligen Trägerstoffe, einem oder mehr Verdickungsmitteln, einem oder mehr oberflächenaktive Mitteln und einem oder mehr Konservierungsmittel ausgewählt werden.

7. Pharmazeutische Zusammensetzung nach Anspruch 6, **dadurch gekennzeichnet, daß** sie eine Mischung aus gesättigten C12-C18-Alkoholestern mit Fettsäure, insbesondere Capryl- und Caprinsäure, als erweichenden Mittel enthält.

8. Pharmazeutische Zusammensetzung nach Anspruch 7, **dadurch gekennzeichnet, daß** sie das erweichende Mittel in einer Konzentration zwischen 2,0% und 20,0% der Masse der Zusammensetzung enthält, noch vorzugsweise zwischen 5,0% und 10,0% der Masse der Zusammensetzung enthält.

9. Pharmazeutische Zusammensetzung nach Anspruch 6, **dadurch gekennzeichnet, daß** sie eine Mischung aus zyklische C14-C18-Kohlenwasserstoffe und aliphatischen C14-C18-Kohlenwasserstoffe, als öligen Trägerstoff enthält.

10. Pharmazeutische Zusammensetzung nach Anspruch 9, **dadurch gekennzeichnet, daß** sie den öligen Trägerstoff in einer Konzentration zwischen 1,0% und 10,0% der Masse der Zusammensetzung enthält, noch vorzugsweise zwischen 2,0% und 5,0% der Masse der Zusammensetzung enthält.

11. Pharmazeutische Zusammensetzung nach Anspruch 6, **dadurch gekennzeichnet, daß** sie eine Mischung aus aliphatischen Alkohole, hauptsächlich Stearyl- und Cetylalkohol, als Verdickungsmittel enthält.

12. Pharmazeutische Zusammensetzung nach Anspruch 11, **dadurch gekennzeichnet, daß** sie das Verdickungsmittel in einer Konzentration zwischen 1,0% und 10,0% der Masse der Zusammensetzung enthält, noch vorzugsweise zwischen 3,0% und 8,0% der Masse der Zusammensetzung enthält.

13. Pharmazeutische Zusammensetzung nach Anspruch 6, **dadurch gekennzeichnet, daß** sie nichtionogen oberflächenaktive Mitteln als Mitteln für die Oberflächenspannung modifizieren, enthält, welche Estern aus gesättigten Fettsäure mit Polyethoxylierten Sorbit und/oder ihren Anhydride umfaßen.

14. Pharmazeutische Zusammensetzung nach Anspruch 13, **dadurch gekennzeichnet, daß** sie das Mittel für die Oberflächenspannung modifizieren in einer Konzentration zwischen 1,0% und 10,0% der Masse der Zusammensetzung enthält, noch vorzugsweise zwischen 2,0% und 5,0% der Masse der Zusammensetzung enthält.

15. Pharmazeutische Zusammensetzung nach Anspruch 6, **dadurch gekennzeichnet, daß** sie Methyl-p-hydroxybenzoat und/oder Propyl-p-hydroxybenzoat als Konservierungsmitteln enthält.

16. Pharmazeutische Zusammensetzung nach irgendeine der Ansprüche 2 bis 15, **dadurch gekennzeichnet, daß** sie als einer Öl-in-Wasser (O/W) Emulsion vorliegt.

17. Pharmazeutische Zusammensetzung nach irgendeine der Ansprüche 2 bis 16, **dadurch gekennzeichnet, daß** sie einen pH-Wert, der sich zwischen 5 und 7,5 ändert, noch vorzugsweise zwischen 5,5 und 7,5 ändert, hat.

18. Pharmazeutische Zusammensetzung nach irgendeine der Ansprüche 2 bis 17, **dadurch gekennzeichnet, daß** sie um 25°C, eine Viskosität, die sich zwischen 100 mPas und 20 000 mPas ändert, noch vorzugsweise zwischen 350 mPas und 5000 mPas ändert, hat.

19. Pharmazeutische Zusammensetzung nach Anspruch 2, **dadurch gekennzeichnet, daß** sie eine Physikalischechemische Stabilität während einer minimale Zeitspanne von 2 Jahre hat.

20. Verfahren zur Herstellung einer stabile und für Hautanwendung geeignete Zubereitung aus Ubidecarenon mit Dexpanthenol und Chlorhexidin oder einem pharmazeutischen annehmbaren Salz derselben, nach irgendeine der Ansprüche 1 bis 19, **dadurch gekennzeichnet, daß** es die Schritte:
a) die Herstellung einer ölige Phase, in welcher Ubidecarenon um einer Temperatur zwischen 50°C und 60°C gegeben wird;
b) die Herstellung einer wässrige Phase, in welcher Chlorhexidin oder ein pharmazeutische annehmbare Salz derselbe in dem oberflächenaktiven Mittel dispergiert wird und Dexpanthenol in Wasser gelöst wird;
c) die Herstellung einer Emulsion um einer Temperatur zwischen 50°C und 60°C, umfaßt.

21. Zubereitung nach Anspruch 1, um als einem Arzneimittel verwendet werden.

22. Zubereitung nach Anspruch 1, um bei der Behandlung des akutes Afterriß verwendet werden.

23. Zubereitung nach Anspruch 1, um bei der Behandlung des chronischen Afterriß verwendet werden.

24. Eine stabile und für Hautanwendung geeignete pharmazeutische Zusammensetzung nach irgendeine der Ansprüche 2 bis 19, welche die Zubereitung nach Anspruch 1 enthält, **dadurch gekennzeichnet, daß** sie bei der Behandlung des akutes Afterriß verwendet wird.

25. Eine stabile und für Hautanwendung geeignete pharmazeutische Zusammensetzung nach irgendeine der Ansprüche 2 bis 19, welche die Zubereitung nach Anspruch 1 enthält, **dadurch gekennzeichnet, daß** sie bei der Behandlung des chronisches Afterriß verwendet wird.

26. Verwendung der stabilen und für Hautanwendung geeigneten Zubereitung nach Anspruch 1, bei der Herstellung eines stabiles Arzneimittels für die Behandlung des akutes Afterriß.

27. Verwendung der stabilen und für Hautanwendung geeigneten Zubereitung nach Anspruch 1, bei der Herstellung eines stabiles Arzneimittels für die Behandlung des chronisches Afterriß.

28. Verwendung der stabilen und für Hautanwendung geeigneten Zubereitung nach Anspruch 1, bei der Herstellung eines stabiles Arzneimittels für die Vorbeugung des Rückfalls des akutes Afterriß.

29. Verwendung der stabilen und für Hautanwendung geeigneten Zubereitung nach Anspruch 1, in Verbindung mit einem Modulator der ionischen Kanäle, bei der Herstellung eines Arzneimittels für die Behandlung des chronisches Afterriß.

30. Verwendung der stabilen und für Hautanwendung geeigneten Zubereitung nach Anspruch 1, in Verbindung mit einem Modulator der ionischen Kanäle, bei der Herstellung eines stabiles Arzneimittels für die Vorbeugung des Rückfalls des chronisches Afterriß.

## Revendications

1. Une combinaison, stable et convenable pour l'application cutanée, d'ubidecarenone avec du dexpanthénol et de la chlorhexidine, ou des sels pharmaceutiquement acceptables des mêmes, **caractérisée en ce qu'**elle se présente sous la forme d'une émulsion huile-en-eau, contenant l'ubidecarenone dissoute dans la phase huile, la chlorhexidine en suspension dans un agent surfactant non-ionique, et le dexpanthénol dissous dans la phase aqueuse.

2. Une composition pharmaceutique, stable et convenable pour l'application cutanée, comprenant la combinaison selon la revendication 1 et des excipients topiques et/ou des adjuvants pharmaceutiquement acceptables.

3. Une composition pharmaceutique selon la revendication 2, **caractérisée en ce que** l'ubidecarenone est présente dans une concentration entre 0,1% et 15% de la masse de la composition, plus préférablement entre 0,5% et 7,5% de la masse de la composition.

4. Une composition pharmaceutique selon la revendication 2, **caractérisée en ce que** le dexpanthénol est présent dans une concentration entre 1% et 15% de la masse de la composition, plus préférablement entre 0,5% et 7,5% de la masse de la composition.

5. Une composition pharmaceutique selon la revendication 2, **caractérisée en ce que** la chlorhexidine est présente dans une concentration entre 0,1% et 5% de la masse de la composition, plus préférablement entre 0,1% et 1% de la masse de la composition.

6. Une composition pharmaceutique selon la revendication 2, **caractérisée en ce que** les excipients et/ou les adjuvants pharmaceutiquement acceptables sont choisis parmi un ou plusieurs émollients, un ou plusieurs matériaux de support huileux, un ou plusieurs épaississants, un ou plusieurs agents surfactants et un ou plusieurs agents conservateurs.

7. Une composition pharmaceutique selon la revendication 6, **caractérisée en ce qu'**elle contient comme émollient un mélange d'esters d'alcools saturés, entre C12 et C18, avec des acides gras, notamment les acides caprylic et caprique.

8. Une composition pharmaceutique selon la revendication 7, **caractérisée en ce que** l'émollient est présent dans une concentration entre 2,0% et 20,0% de la masse de la composition, plus préférablement entre 5,0% et 10,0% de la masse de la composition.

9. Une composition pharmaceutique selon la revendication 6, **caractérisée en ce qu'**elle contient comme un matériau de support huileux un mélange d'hydrocarbures cycliques et d'hydrocarbures aliphatiques, entre C14 et C18.

10. Une composition pharmaceutique selon la revendication 9, **caractérisée en ce que** le matériau de support huileux est présent dans une concentration entre 1,0% et 10,0% de la masse de la composition, plus préférablement entre 2,0% et 5,0% de la masse de la composition.

11. Une composition pharmaceutique selon la revendication 6, **caractérisée en ce qu'**elle contient comme un épaississant un mélange d'alcools aliphatiques, surtout l'alcool stéarylique et l'alcool cétylique.

12. Une composition pharmaceutique selon la revendication 11, **caractérisée en ce que** l'épaississant est présent dans une concentration entre 1,0% et 10,0% de la masse de la composition, plus préférablement entre 3,0% et 8,0% de la masse de la composition.

13. Une composition pharmaceutique selon la revendication 6, **caractérisée en ce qu'**elle contient comme des agents de modification de la tension superficielle des agents surfactants non-ioniques qui sont compris parmi les esters d'acides gras avec le sorbitol polyéthoxylé et/ou leurs anhydrides.

14. Une composition pharmaceutique selon la revendication 13, **caractérisée en ce que** l'agent de modification de la tension superficielle est présent dans une concentration entre 1,0% et 10,0% de la masse de la composition, plus préférablement entre 2,0% et 5,0% de la masse de la composition.

15. Une composition pharmaceutique selon la revendication 6, **caractérisée en ce qu'**elle contient comme des agents conservateurs le parahydroxybenzoate de méthyle et/ou le parahydroxybenzoate de propyle ou le propylène glycol.

16. Une composition pharmaceutique selon l'une quelconque des revendications 2 à 15, **caractérisée en ce qu'**elle se présente sous la forme d'une émulsion huile-en-eau (H/E).

17. Une composition pharmaceutique selon l'une quelconque des revendications 2 à 16, **caractérisée en ce qu'**elle a un pH qui varie entre 5 et 7,5, plus préférablement entre 5,5 et 7,5.

18. Une composition pharmaceutique selon l'une quelconque des revendications 2 à 17, **caractérisée en ce qu'**elle a une valeur pour la viscosité qui varie entre 100 mPas et 20 000 mPas, plus préférablement entre 350 mPas et 5000 mPas, à 25°C.

19. Une composition pharmaceutique selon la revendication 2, **caractérisée en ce qu'**elle a une stabilité physico-chimique pendant une période de temps minimum de 2 années.

20. Procédé pour la préparation d'une combinaison, stable et convenable pour l'application cutanée, d'ubidecarenone avec du dexpanthénol et de la chlorhexidine, ou des sels pharmaceutiquement acceptables des mêmes, selon l'une quelconque des revendications 1 à 19, **caractérisé en ce qu'**il comprend les étapes de:
a) la préparation d'une phase huile à laquelle l'ubidecarenone est ajoutée à une température entre 50°C et 60°C;
b) la préparation d'une phase aqueuse, dans laquelle la chlorhexidine, ou un sel pharmaceutiquement acceptable de la même, est dispersée dans l'agent surfactant et le dexpanthénol est dissous dans l'eau;
c) la préparation d'une émulsion à une température entre 50°C et 60°C.

21. La combinaison selon la revendication 1, pour être utilisée comme un médicament.

22. Une combinaison selon la revendication 1, pour être utilisée dans le traitement de la fissure anale aigue.

23. Une combinaison selon la revendication 1, pour être utilisée dans le traitement de la fissure anale chronique.

24. Une composition pharmaceutique, stable et convenable pour l'application cutanée, selon l'une quelconque des revendications 2 à 19, contenant la combinaison selon la revendication 1, **caractérisé en ce qu'**elle est utilisée dans le traitement de la fissure anale aigue.

25. Une composition pharmaceutique, stable et convenable pour l'application cutanée, selon l'une quelconque des revendications 2 à 19, contenant la combinaison selon la revendication 1, **caractérisé en ce qu'**elle est utilisée dans le traitement de la fissure anale chronique.

26. L'utilisation de la combinaison stable et convenable pour l'application cutanée, selon la revendication 1, dans la préparation d'un médicament stable pour le traitement de la fissure anale aigue.

27. L'utilisation de la combinaison stable et convenable pour l'application cutanée, selon la revendication 1, dans la préparation d'un médicament stable pour le traitement de la fissure anale chronique.

28. L'utilisation de la combinaison stable et convenable pour l'application cutanée, selon la revendication 1, dans la préparation d'un médicament stable pour la prévention de la récidive de la fissure anale aigue.

29. L'utilisation de la combinaison stable et convenable pour l'application cutanée, selon la revendication 1, associée à un modulateur des canaux ioniques, dans la préparation d'un médicament pour le traitement de la fissure anale chronique.

30. L'utilisation de la combinaison stable et convenable pour l'application cutanée, selon la revendication 1, associée à un modulateur des canaux ioniques, dans la préparation d'un médicament pour la prévention de la récidive de la fissure anale chronique.
